# EUROPÄISCHE PATENTSCHRIFT

(11) **EP 1 707 230 B1**
(45) Veröffentlichungstag und Bekanntmachung des Hinweises auf die Patenterteilung: **17.02.2016**
(21) Anmeldenummer: 06013171.1
(22) Anmeldetag: 26.02.2002
(51) Int. Cl.: A61M 5/32, A61M 25/06

(54) **Injektionsnadel mit Schutzelement für die Nadelspitze**
Injection needle with a protection element for the needle tip
Aiguille d'injection avec un élément de protection pour la pointe de l'aiguille

(30) Priorität: 26.02.2001 DE 20103363 U
(43) Veröffentlichungstag der Anmeldung: 04.10.2006
(62) Teilanmeldung aus: 02719930.6
(73) Patentinhaber: B. Braun Melsungen AG, 34212 Melsungen (DE)
(72) Erfinder: Woehr, Kevin, 34587 Felsberg (DE); Fuchs, Jürgen, 34308 Bad Emstal (DE)
(74) Vertreter: Kinkeldey, Daniela

(56) Entgegenhaltungen:
- EP-A- 0 554 841
- EP-A- 0 753 317
- WO-A-99/08742
- DE-A1- 4 434 569

## Beschreibung

Die Erfindung betrifft eine Schutzvorrichtung für eine Injektions- bzw. Infusionsnadel nach dem Oberbegriff des Anspruchs 1.

Eine Schutzvorrichtung dieser Art ist aus EP-A-0 554 841 bekannt, wobei ein eine Blattfeder aufnehmendes Gehäuse für den Einsatz in eine Kathetervorrichtung vorgesehen ist. Die Blattfeder weist einen federnden Arm mit einem abgewinkelten Ende zum Übergreifen der Nadelspitze in der Schutzstellung auf und ist über einen Ansatz an der proximalen Rückwand in eine seitliche Öffnung des Gehäuses eingeklemmt. Ein in gleicher Weise ausgebildetes Nadelschutzelement aus einem an den Enden - abgesehen von der Durchtrittsöffnung für die Nadel - geschlossenen Gehäuse mit darin angeordneter Blattfeder ist aus DE 44 34 569 A1 bekannt.

Weiterhin beschreibt EP-A-0 753 317 ein Nadelschutzelement mit einem äußeren Gehäuse, das an den Enden geschlossen ist und ein federbeaufschlagtes Innengehäuse umgibt, in dem ein kippbares Metallelement zum Abdecken der Nadelspitze in der Schutzstellung angeordnet ist.

Eine weitere Schutzvorrichtung ist aus US 4 929 241 bekannt, wobei ein relativ kleines Schutzelement auf der Nadel angeordnet ist, das mittels einer Feder aus der zurückgezogenen Stellung in die Schutzstellung an der Nadelspitze verschoben werden kann, wobei elastische Arme des Schutzelementes die Nadelspitze übergreifen, während eine Eingriffseinrichtung am Schutzelement dieses auf dem Nadelschaft hält. Aufgrund der geringen Größe des Schutzelementes ist es schwierig, dieses von Hand auf der Nadel zu verschieben. Zudem kann die Befestigungsfeder erst dann ausgelöst werden, wenn die Nadelspitze frei liegt, so dass eine Verletzungsgefahr nicht ausgeschlossen werden kann.

Der Erfindung liegt die Aufgabe zugrunde, eine Schutzvorrichtung der eingangs angegebenen Art so auszubilden, dass eine Betätigung von Hand erleichtert wird und eine Verletzungsgefahr ausgeschlossen werden kann.

Diese Aufgabe wird erfindungsgemäß durch die Merkmale im kennzeichnenden Teil des Anspruchs 1 gelöst. Dadurch, dass ein Griffteii, das einen Hohlraum mit offenem Ende fur Aufnahme des Schutzelementes aufweist, zwischen Schutzelement und Nadelhalter zum Verschieben des Schutzelementes vorgesehen ist, kann beim Zurückziehen der Nadel mittels des Nadelhalters nach einer Injektion mit der anderen Hand das Griffteil bequem gehalten werden so dass durch die Relativbewegung zwischen Nadel und Griffteil die zweigegenüberliegenden federudem Arme des Schutzelementes in die Schutzposition an der Nadelspitze verschoben werdem und dabei die Nadelspitze übergreifen, ohne dass das kleine Schutzelement mit den Fingern berührt werden muss. Dadurch, dass das Schutzelement während des Zurückziehens der Nadel in die Schutzposition gebracht wird, kann auch eine Verletzungsgefahr ausgeschlossen werden.

Beispielsweise Ausführungsformen der Erfindung werden nachfolgend anhand der Zeichnungen näher erläutert Es zeigen
- Fig. 1: in einem Längsschnitt eine Schutzvorrichtung,
- Fig. 2: eine Seitenansicht der Ausführungsform nach Fig. 1
- Fig. 3: eine Ansicht der Vorrichtung nach den Fig. 1 und 2 mit in die Schutzstellung verschobenem Schutzelement,
- Fig. 4: eine andere Ausführungsform eines Griffteiles in Verbindung mit einer Spritze,
- Fig. 5: eine Ausführungsform in Verbindung mit einem mit Flügeln versehenen Nadelhalter,
- Fig. 6: eine Schnittansicht der Ausführungsform nach Fig. 5,
- Fig. 7: eine Stirnansicht in Fig. 5,
- Fig. 8: eine Draufsicht auf eine Ausführungsform nach Fig. 5 mit Nadelkappe,
- Fig. 9: eine Ansicht der Nadelkappe nach Fig. 8,
- Fig. 10: eine Draufsicht auf eine Ausfuhrungsform nach Fig. 5 mit einer abgewandelten Nadelkappe,
- Fig. 11: eine Seitenansicht der Nadelkappe nach Fig. 10,
- Fig. 12: einen Längsschnitt durch eine Nadelkappe,
- Fig. 13: eine Schnittansicht durch eine andere Ausführungsform mit gekrümmter Nadel,
- Fig. 14: eine andere Nadelkappe bei der Ausführungsform nach Fig. 13,
- Fig. 15: eine Ausführungsform mit verformbarem Griffteil in der Ausgangsstellung, und
- Fig. 16: das Griffteil von Fig. 15 in der gestreckten Stellung.

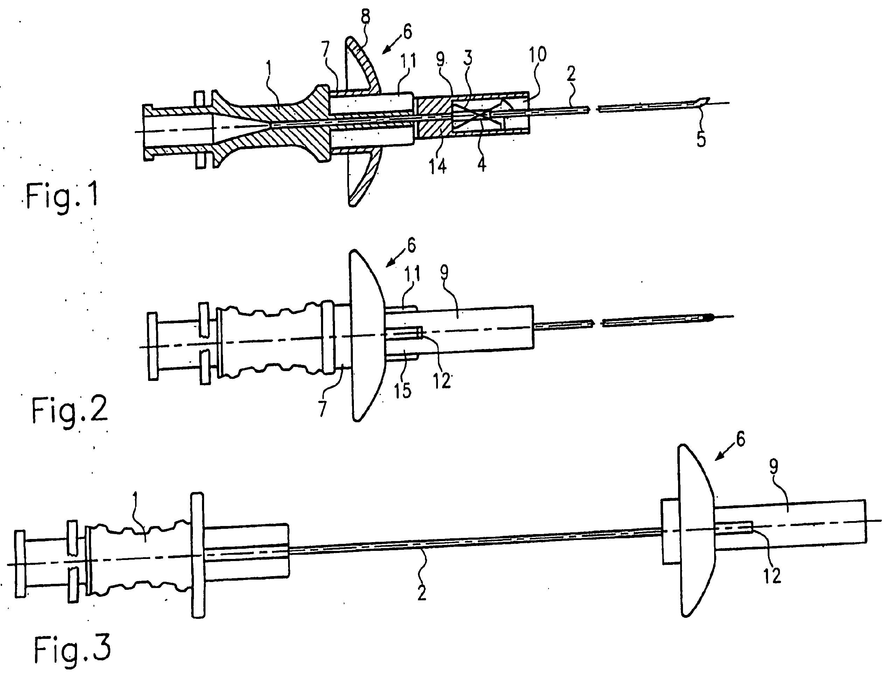

Fig. 1 zeigt einen Nadelhalter 1, in dem eine Nadel 2 befestigt ist. Auf dem Schaft der Nadel 2 ist ein Schutzelement 3 in Form eines Federclips mit sich kreuzenden Armen angeordnet. Mit 4 ist eine Hülse bezeichnet, die mit dem Schutzelement 3 längs des Nadelschaftes verschiebbar ist. Bei dem dargestellten Ausführungsbeispiel ist die Spitze 5 der Nadel entsprechend einer Epidural-Nadel oder einer Huber-Nadel gekrümmt ausgebildet, so dass die Hülse 4, die einen kleineren Durchmesser als die Krümmung an der Nadelspitze aufweist, und mit ihr das Schutzelement 3 nicht über die Nadelspitze hinaus verschoben werden kann.

Zwischen Nadelhalter 1 und Schutzelement 3 ist ein Griffteil 6 angeordnet, das am proximalen Ende einen hohlzylindrischen Abschnitt 7 aufweist, an dem ein radial abstehender Schild 8 ausgebildet ist. Auf der Vorderseite des Schildes 8 ist ein zylindrischer Abschnitt 9 ausgebildet, dessen distales Ende hohl ausgebildet ist. In dem Hohlraum 10 ist in der Bereitstellung nach Fig. 1 das Schutzelement 3 angeordnet, das durch Verschieben des Griffteiles 6 nach vorne zur Nadelspitze 5 verschoben werden kann, während mit der anderen Hand der Nadelhalter 1 gehalten wird. Hierbei übergreifen die abgewinkelten Enden der sich kreuzenden Arme des Schutzelementes 3 die Nadelspitze 5, so dass eine Verletzung der Bedienungsperson durch die Nadelspitze verhindert wird.

Der Nadelhalter 1 weist am distalen Ende radial abstehende Rippen 11 auf, auf denen der hohlzylindrische Abschnitt 7 des Griffteiles 6 geführt ist Zwischen dem zylindrischen Abschnitt 9 mit kleinerem Außendurchmesser und dem hohlzylindrischen Abschnitt 7 mit größerem Außendurchmessers sind in dem Griffteil 6 Schlitze 12 ausgebildet, durch welche die vorderen Enden der Rippen 11 des Nadelhalters 1 radial vorstehen, wie Fig. 2 zeigt.

Der mit dem Hohlraum 10 versehene zylindrische Abschnitt 9 des Griffteils 6 weist einen vollzylindrischen Abschnitt 14 zwischen den Schlitzen 12 und dem Hohlraum 10 auf, in dessen zentrischer Bohrung die Nadel 2 geführt ist. Zwischen den Schlitzen 12 des Griffteils 6 ist der zylindrische Abschnitt 9 über Stege 15 mit dem Schild 8 bzw. dem hohlrylindrischen Abschnitt 7 einstückig verbunden.

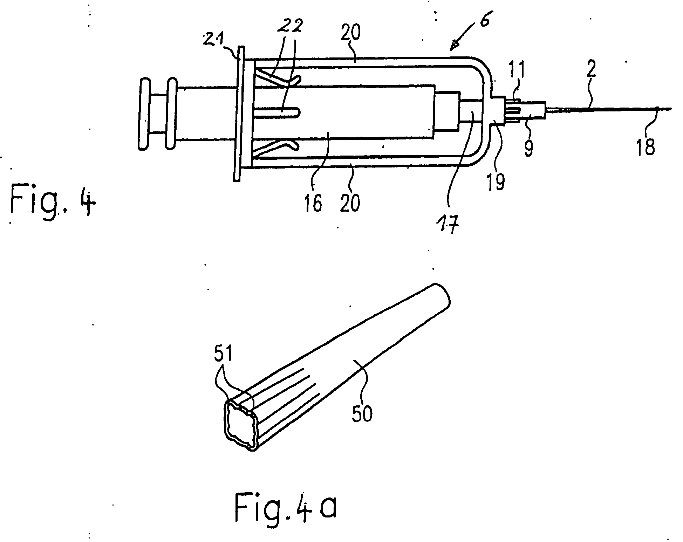

Diese über den Außenumfang des zylindrischen Abschnittes 9 des Griffteiles 6 vorstehenden Rippen 11 dienen zum Aufstecken einer Nadelkappe 13, die in Fig. 4 gezeigt ist. Diese Nadelkappe 13 wird für die Lagerung und die Handhabung der Vorrichtung verwendet. Sie kann unmittelbar vor Gebrauch der Injektionsnadel vom Nadelhalter 1 abgezogen werden, um die Nadel.freizulegen, ohne dass dadurch das Griffteil 6 und das Schutzelement 3 bewegt wird, weil die Nadelkappe 13 durch die Rippen 11 in einem radialen Abstand von dem Abschnitt 9 des Griffteils 6 gehalten wird.

Aufgrund des kleineren Durchmessers am Abschnitt 14 gegenüber dem größeren Durchmesser an den Rippen 11 kann die Nadelkappe 13, die aus einem Schlauchstück mit durchgehend gleichem Durchmesser besteht, nicht auf dem Abschnitt 14 fehlpositioniert, sondern nur auf die Rippen 11 aufgesteckt werden. Hierdurch wird verhindert, dass ein Eingriff der Nadelkappe 13 mit einem Abschnitt des Griffteils 6 unbeabsichtigt hergestellt wird. Die Nadelkappe 13 kann durch Extrudieren eines Schlauches kostengünstig gefertigt werden, wobei ein Stück eines solchen Schlauches die Nadelkappe 13 bildet.

Nach Abnehmen der Nadelkappe 13 kann in der Bereitstellung nach den Fig. 1 und 2 eine Injektion ausgeführt werden, worauf beim Zurückziehen der Nadel mit einer Hand am Nadelhalter 1 mit der anderen Hand das Griffteil 6 am Abschnitt 7 gehalten wird, so dass durch die Relativbewegung zwischen Griffteil 6 und Nadel 2 das Schutzelement 3 in die Schutzstellung an der Nadelspitze verschoben wird. Diese ausgefahrene Stellung des Griffteiles 6 ist in Fig. 3 wiedergegeben.

Das Schutaelement 3 ist in dem Hohlraum 10 des Griffteils 6 lose angeordnet, so dass das Griffteils 6 aus der Stellung in Fig. 3 ohne weiteres zurückgezogen werden kann, während das Schutzelement in der Schutzstellung an der Nadelspitze verbleibt Der Hohlraum 10 im zylindrischen Abschnitt 9 schützt das Schutzelement 3 nach Abziehen der Nadelkappe 13.

Bei der Ausführungsform nach Fig. 1 und 2 dient der hohlzylindrische Abschnitt 7 am Griffteil 6 dazu, die Finger der das Griffteil haltenden Hand vor einer Berührung mit dem Nadelschaft zu schützen, wenn die Nadel zurückgezogen wird.

Bei einer anderen Ausgestaltung des Nadelhalters 1 kann dieser hohlzylindrische Abschnitt 7 hinter dem Schild 8 vergrößert ausgebildet sein.

Das Griffteil 6 wird - wie auch der Nadelhalter 1 - zweckmäßigerweise aus Kunststoff gefertigt.

Fig. 4 zeigt eine abgewandelte Ausführungsform eines Griffteils 6 in Verbindung mit einer Spritze 16, an der über einen Nadelhalter 17, der als Kanülenansatz ausgebildet ist, eine Injektionsnadel 2 fest angebracht ist. Bei dieser Ausführungsform ist vor der Nadelspitze ein Wulst 18 auf dem Außenumfang der Nadel ausgebildet, an dem die Rückwand des Schutzelementes 3 in der Schutzstellung zum Anliegen kommt. Anstelle eines Wulstes 18 können durch eine Nadelquetschung auch diametral gegenüberliegende noppenartige Vorsprünge ausgebildet werden.

Das Griffteil 6 weist einen zylindrischen Abschnitt 19 auf, der in der Ausgangsstellung nach Fig. 4 auf dem Nadelhalter 17 geführt ist Von diesem zylindrischen Abschnitt 19 erstrecken sich bei dem dargestellten Ausführungsbeispiel an diametral gegenüberliegenden Stellen zwei Bügel 20 in einem Abstand vom Spritzenumfang in proximale Richtung. Die Enden dieser Bügel 20 sind an einem Ringkörper 21 angeformt, von dem aus sich elastische Finger 22 radial nach innen erstrecken. Die freien Enden dieser elastischen Finger 22 liegen auf dem Außenumfang der Spritze 16 an.

Aufgrund der elastischen Finger 22 zwischen Griffteil 6 und Außenumfang der Spritze 16 kann das Griffteil 6 für unterschiedlich große Spritzendurchmesser verwendet werden, z. B. können Spritzen mit einem Volumen von 1 ml bis 10 ml in das gleiche Griffteil eingesetzt werden. Hierdurch ist eine große Auswahl von Spritzen möglich, die mit der gleichen Nadel verwendet werden können.

Auch bei der Ausführungsform nach Fig. 4 sind am vorderen Ende des als Kanülenansatz ausgebildeten Nadelhalters radial abstehende Rippen 11 ausgebildet werden, die als Sitz für eine Nadelkappe dienen. Durch den Innenumfang des zylindrischen Abschnitts 19 wird das Schutzelement 3, dessen Rückwand über den Querschnitt der Rippen 11 vorsteht, nach vorne in die Schutzstellung verschoben.

An dem zylindrischen Abschnitt 19 ist ein weiterer zylindrischer Abschnitt 9 angeformt ist, in dessen Hohlraum 10 das Schutzelement 3 aufgenommen wird. Wie bei der Ausführungsform nach den Fig. 1 und 2 sind zwischen zylindrischem Abschnitt 19 und Abschnitt 9 in Achsrichtung verlaufende Schlitze ausgebildet, durch die die am Nadelhalter bzw. Kanülenansatz 17 ausgebildeten Rippen 11 zur Aufnahme der Nadelkappe 13 vorstehen.

Fig. 4a zeigt in perspektivischer Ansicht eine durch Spritzgießen ausgebildete Nadelkappe 50, deren distales Ende geschlossen sein kann, während das proximale Ende auf dem Innenumfang entsprechend der Anzahl der Rippen 11 Rillen oder Nuten 51 aufweist, die beim Aufstecken der Nadelkappe auf den Nadelhalter 17 mit den Rippen 11 in Eingriff treten, so dass durch Verdrehen der aufgesteckten Nadelkappe 50 auch der Nadelhalter 17 verdreht werden kann. Zwischen Nadelhalter 17 und Spritze 16 wird üblicherweise ein Gewindeeingriff vorgesehen, so dass durch Verdrehen der Nadelkappe 50 der Nadelhalter 17 auf die Spritze 16 aufgeschraubt werden kann.

Es ist üblich, mittels einer Nadel mit relativ großem Durchmesser Flüssigkeit in die Spritze anzusaugen und dann die Nadel gegen eine Nadel mit einem relativ kleinen Durchmesser auszuwechseln, um eine Infusion am Patienten auszuführen. Bei der Ausführungsform nach dem Fig. 4 kann die Nadel ohne weiteres ausgewechselt werden.
Die beschriebene Ausgestaltung ermöglicht eine Betätigung mit einer Hand, wenn der Spritzeninhalt injiziert ist, wobei die Spritze 16 mit zwei Fingern gehalten und die Nadel aus der Haut des Patienten gezogen wird, während gleichzeitig sich ein Finger der Hand an dem am proximalen Ende liegenden Ringkörper 21 abstützt.

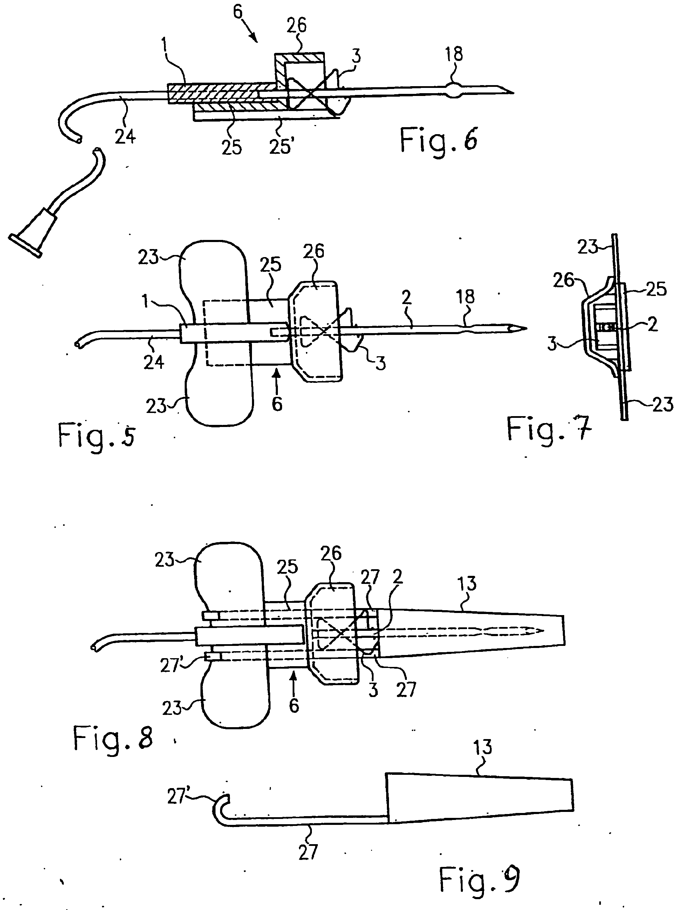

Fig. 5 zeigt in einer Draufsicht einen mit seitlich abstehenden Flügeln 23 versehenen Nadelhalter 1, an den ein Verbindungsschlauch 24 angeschlossen ist. Zwischen dem auf dem Nadelschaft angeordneten Schutzelement 3 und Nadelhalter 1 ist ein Griffteil 6 mit einem haubenförmigen Abschnitt 26 angeordnet, das wegen des flachen Injektionswinkels (Fig. 6) zweckmäßigerweise ein Flächenstück 25 zur Auflage auf der Haut des Patienten aufweist, das auf der Auflageseite beispielsweise mit einer Klebeschicht zur besseren Halterung auf der Haut versehen sein kann. Vorzugsweise wird ein Schaummaterial 25' auf der Auflageseite vorgesehen. Der von dem vorderen Ende des Flächenstücks 25 vorstehende Haubenabschnitt 26 des Griffteils 6 überdeckt wenigstens teilweise das Schutzelement 3. Das Flächenstück 25 bzw. das weiche Auflageteil 25' dient auch als Abstandhalter für das Schutzelement 3 von der Haut des Patienten. Bei dem Ausführungsbeispiel nach Fig. 6 erstreckt sich der weiche Auflageteil 25' über das Flächenstück 25 unter den Haubenabschnitt 26, so dass das Schutzelement 3 nicht auf der Haut des Patienten aufliegt.

Der mit Flügeln 23 versehene Nadelhalter 1 wird für Veneninfusionen verwendet, wobei üblicherweise eine dünne Nadel verwendet wird. Die Flügel 23 sind relativ groß und flexibel. Sie werden zusammengedrückt, wenn die Nadel in einem sehr flachen Winkel in die Haut eingeführt wird. Ein auf der Klebeschicht an der Auflagefläche aufgebrachtes, nicht dargestelltes Schutzpapier sollte nicht abgezogen werden, bis die Nadel in der Vene eingeführt ist. Nachdem die Nadel in die Vene eingeführt ist, werden die Flügel 23 auf der Haut des Patienten flach gelegt und mit einem Klebeband gehalten. Auch das Griffteil 6 kann mittels eines Klebebandes festgelegt werden, wobei der haubenförmige Abschnitt 26 einen Kontakt zwischen Schutzelement 3 und Klebeband verhindert. Beim Zurückziehen der Nadel nach Entfernen des Klebebandes vom Nadelhalter bleibt zunächst das Griffteil 6 mit dem Schutzelement 3 in seiner Stellung. Nachdem die zurückgezogene Nadelspitze durch das Schutzelement 3 sicher abgedeckt ist, wobei die Vorsprünge 18 an der Nadel das Schutzelement 3 an der Nadelspitze fixieren, kann auch das Griffteil 6 von der Haut des Patienten abgenommen werden.

Fig. 5 und 6 zeigen die Vorrichtung in der Bereitstellung zum Einführen der Nadel. Wenn die mit einer Klebeschicht versehene Auflage 25 am Griffteil verwendet wird, handelt es sich um ein passives System.

Fig. 7 zeigt eine Ansicht des Griffteils 6 von rechts in Fig. 5 Die Flügel 23 dienen als Auflagefläche für den Nadelhalter 1, nachdem die Infusionsnadel eine gewisse Zeit in der eingefübrten Stellung verbleiben muss.

Fig. 8 und 9 zeigen bei einem Aufbau nach den Fig. 5 bis 7 eine Nadelkappe 13, die mit zwei beabstandeten Haltebügeln 27 versehen ist; die über ein abgebogenes freies Ende 27' an den Flügeln 23 eingehängt werden, wie Fig. 8 zeigt. Bei dieser Ausführungsform liegt das proximale Ende der Nadelkappe 13 zweckmäßigerweise am Vorderende des Schutzelementes 3 an, wie Fig. 8 zeigt, damit das Schutzelement 3 in seiner Bereitstellung gehalten wird.

Es ist aber auch möglich, am proximalen Ende der Nadelkappe 13 einen haubenförmigen Ansatz vorzusehen, der auf der Vorderseite des haubenförmigen Abschnitts 26 anliegt.

Bei den beschriebenen Ausführungsformen ist jeweils ein Schutzelement in der Form eines Federclips mit sich kreuzenden Armen wiedergegeben. Es kann aber auch eine andere Ausführungsform eines Schutzelementes in Verbindung mit dem Griffteil 6 verwendet werden.

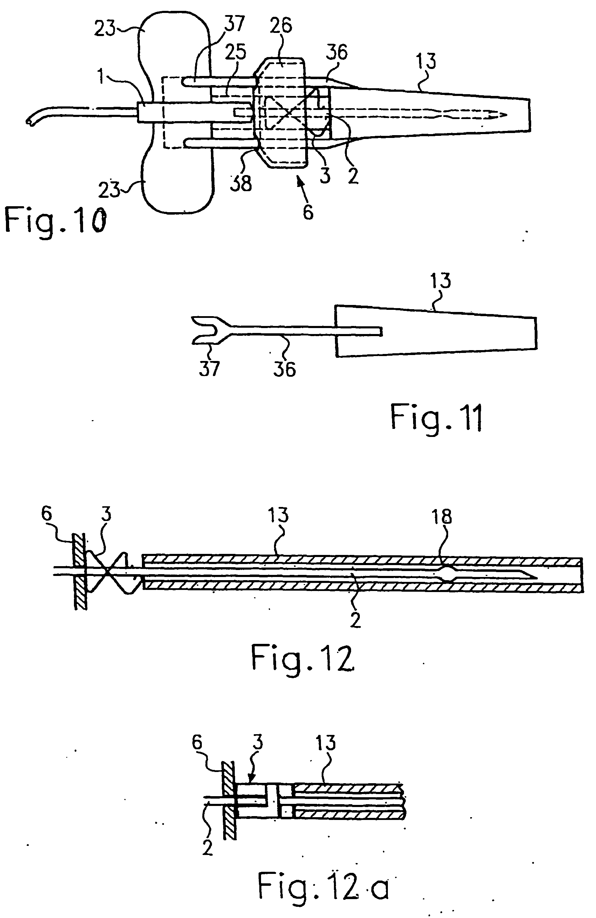

Fig. 10 zeigt eine weitere Ausführungsform der Vorrichtung nach den Fig. 5 bis 7. Die Nadelkappe 13 ist hierbei anstelle der Einhängebügel 27 auf den beiden Seiten mit jeweils einer Verlängerungsstrebe 36 versehen, die am freien Ende einen gabelförmigen Abschnitt 37 zum Einstecken an den Flügeln 23 des Nadelhalters aufweist (Fig. 11). Diese beiden beabstandeten Streben 36 erstrecken sich durch entsprechend groß ausgebildete Öffnungen 38 in dem Hanbenabschnitt 26 des Griffteils 6, so dass die gabelförmigen Steckabschnitte 37 ohne weiteres durch diese Öffnungen 38 abgezogen werden können. Beim Abziehen der Nadelkappe 13 wird der Nadelhalter 1 gehalten, wobei das Griffteil 6 nicht bewegt wird.

Fig. 12 zeigt einen Längsschnitt durch eine Nadelkappe 13, deren proximales Ende am Schutzelement 3 anliegt. Die Nadelkappe ist rohrförmig ausgebildet, wobei die durch Quetschung ausgebildete Durchmesservergrößerung 18 an der Nadel 2 als Abstandhalter für die Nadelkappe 13 dient. Eine solche Nadelkappe kann durch Extrudieren oder Spritzgießen hergestellt werden. Es ist auch möglich, auf dem Innenumfang der Nadelkappe einen Wulst oder Noppen anzuformen, die am Nadelschaft aufliegen und die Nadel im Wesentlichen konzentrisch in der Nadelkappe führen. Die Nadelkappe 13 wird hierbei durch Reibung an den Vorwölbungen 18 auf der Nadel 2 gehalten.

Nach einer weiteren Ausgestaltung kann die Nadelkappe nach dem Aufstecken auf die Nadel durch Wärme und Druck bzw. durch Schrumpfen auf der Nadel festgelegt werden.

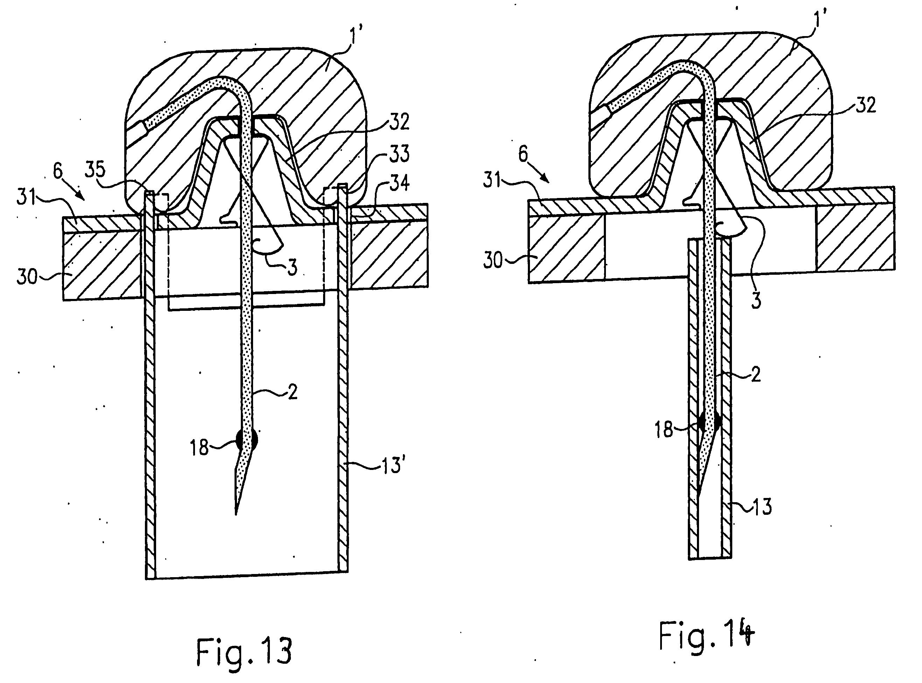

Fig. 13 zeigt eine Ausgestaltung in Verbindung mit einer Huber-Nadel 2, die mittels eines abgebogenen Abschnitts in einem Nadelhalter 1' gehalten ist und zum senkrechten Einführen bei einer Injektion vorgesehen ist. Mit 30 ist ein vorzugsweise aus Schaumstoffmaterial bestehendes Auflageteil bezeichnet, das mit einer Klebefläche zur besseren Fixierung auf der Haut des Patienten versehen ist. Zwischen dem Auflageteil 30 und dem Nadelhalter 1' ist ein schildförmiges Griffteil 6 angeordnet, das über einen flanschartigen Bereich 31 auf dem Auflageteil 30 aufliegt und sich über einen topfförmigen Mittelabschnitt 32 in eine entsprechende Vertiefung des Nadelhalters 1' erstreckt. In diesem topfförmigen Mittelteil 32 ist das Schutzelement 3 angeordnet.

Beim Herausziehen der Nadel wird das Griffteil 6 auf dem Auflageteil 30 gehalten, während der Nadelhalter 1' abgezogen wird. Hierbei wird das Schutzelement 3 zur Nadelspitze verschoben, bis es an der Nadelkräpfung 18 zum Anliegen kommt, während gleichzeitig die beiden sich kreuzenden Arme des Schutzelementes 3 die Nadelspitze übergreifen und abdecken. Hierauf kann das Griffteil 6 vom Auflageteil 30 öder zusammen mit diesem abgenommen werden. Griffteil 6 und Auflageteil 30 können über eine Klebeschicht auch miteinander verbunden sein.

Die Seitenwände des topfförmigen Mittelteils 32 sind vorzugsweise kegelförmig geneigt, so dass das Griffteil 6 selbst nicht abgezogen, sondern nur gedrückt werden kann.

Fig. 13 zeigt eine Nadelkappe 13' mit einem rohrförmigen Abschnitt, an dessen proximalem Ende diametral gegenüberliegende Wandabschnitte 33 vorstehen, die. über teilkreisförmige Schlitze 34 in dem Flansch 31 des Griffteils 6 in entsprechend teilkreisförmige Nuten 35 im Nadelhalter 1' eingesteckt sind. Die gebogenen Wandabschnitte 33 sind lose durch die gebogenen Schlitze 34 im Flansch 31 des Griffteils 6 geführt und mit Preßsitz in die Nuten 35 des Nadelhalters 1' eingesteckt.

Wie bei den anderen Ausführungsformen einer Nadelkappe 13 kann auch die Nadelkappe 13' in Fig. 13 an der distalen Seite geschlossen ausgebildet sein.

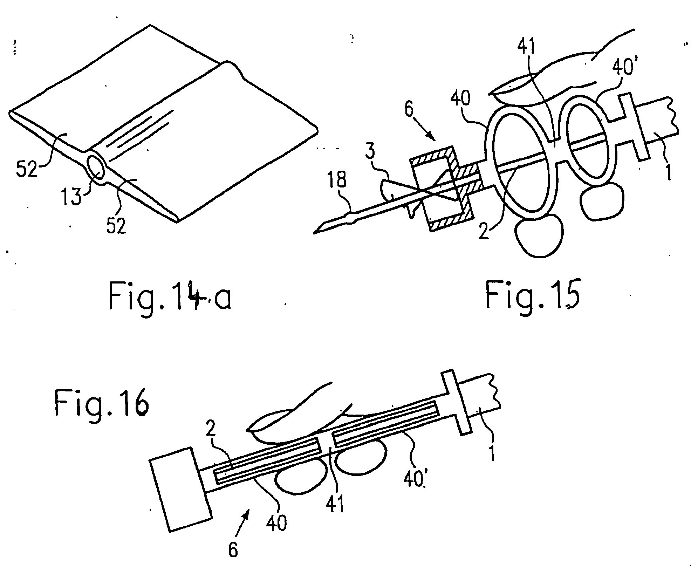

Fig. 14 zeigt eine Ausgestaltung mit Huber-Nadel 2 entsprechend Fig. 13, wobei eine Nadelkappe 13 mit kleinerem Innendurchmesser auf die Nadel 2 aufgeschoben ist. Die Nadelkappe entspricht im Wesentlichen der nach Fig. 13, wobei die Nadelkappe 13 durch Reibung an dem abgewinkelten Vorderende auf der Nadel gehalten wird. Diese Nadelkuppe 13 in Fig. 14 kann mit radial abstehenden und diametral gegenüberliegenden Flächenabschnitten 52 versehen sein, durch welche die Handhabung verbessert und die schlauchförmige Nadelkappe 13 versteift wird. Fig. 14a zeigt in perspektivischer Ansicht eine solche Nadelkappe 13 mit diametral gegenüberliegenden Flächenabschnitten 52.

Fig. 15 und 16 zeigen eine Ausführungsform, bei der das Griffteil 6 einen verformbaren Abschnitt aufweist, mittels dem das distale Ende des Griffteils, an dem das Schutzelement 3 anliegt, in Richtung der Schutzposition an der Nadelspitze verschoben werden kann, indem der verformbare Abschnitt verformt wird. Bei dem Ausführungsbeispiel nach Fig. 15 und 16 sind zwei Paare von formbaren Bügeln 40 und 40' an dem Griffteil 6 ausgebildet, die mit den Fingern zusammengedrückt werden können, so dass sie aus der gebogenen Stellung in Fig. 15 in eine gestreckte Stellung in Fig. 16 überführt werden. Die beiden verformbaren Bügelpaare 40 und 40' sind durch einen Hülsenabschnitt 41 miteinander verbunden. Es ist auch möglich, zwischen den beiden Bügelpaaren 40 und 40' ein Element einzusetzen, das durch Druck mittels der Finger die beiden verformbaren Bügel 40 und 40' in die gestreckte Stellung nach Fig. 15 überführt.

Die Ausführungsformen nach den Fig. 1 bis 14 haben den Vorteil, dass eine größere Kanülenlänge in der Bereitstellung zur Verfügung steht, weil das Schutzelement 3 unmittelbar am Nadelhalter anliegt, während bei den Ausführungsformen nach den Fig. 15 und 16 ein aufwendigerer Aufbau des Griffteils 6 zwischen Schutzelement 3 und Nadelhalter 1 vorgesehen ist, wodurch die zur Verfügung stehende Kanülenlänge eingeschränkt wird.

Bei allen Ausführungsformen wird als Schutzelement 3 bevorzugt ein Nadelclip aus Metall verwendet, dessen sich kreuzende Arme von gegenüberliegenden Seiten eines proximalen Wandabschnitts ausgehen, der ein Loch für.den Durchtritt der Nadel aufweist, wobei der Lochdurchmesser kleiner ist als die maximale Querabmessung der Nadel an der Quetschung 18, so dass der Nadelclip durch den im Durchmesser vergrößerten Abschnitt 18 in der Schutzstellung an der Nadelspitze gehalten wird. Die sich kreuzenden Arme, die beiderseits der Nadel 2 verlaufen, wie Fig. 12a zeigt, weisen am distalen Ende einen etwa auf die Breite der Rückwand verbreiterten Endabschnitt auf, der in der Ausgangsstellung auf dem Außenumfang der Nadel unter elastischer Vorspannung aufliegt und bei Erreichen der Nadelspitze durch Federwirkung in die Schutzstellung bewegt wird, in der die beiden verbreiterten Endabschnitte die Nadelspitze übergreifen. Hierzu sind die distalen Enden der Arme, wie die Seitenansichten zeigen, in Längsrichtung etwas versetzt zueinander bzw. die Arme unterschiedlich lang, so dass sichergestellt ist, dass die beiden abgewinkelten Endabschnitte der Arme die Nadelspitze übergreifen. Zumindest am längeren Arm ist der Endabschnitt am freien Rand nach innen gebogen, um die Abdeckung der Nadelspitze auch dann zu gewährleisten, wenn versucht werden sollte, den Nadelclip aus der Schutzstellung auf der Nadel zurückzuschieben, wobei sich der nach innen abgebogene Endabschnitt an der Nadelspitze verhakt. Der Nadelclip kann insgesamt sehr kompakt ausgebildet werden und nur etwa 7 mm lang sein.

## Patentansprüche

1. Schutzvorrichtung für eine Injektions- bzw. Infusionsnadel, umfassend
- einen Nadelhalter (1) am proximalen Ende der Nadel
- eine vorspringende Eingriffseinrichtung (18) am Nadelumfang nahe der Nadelspitze,
- ein Schutzelement (3) für die Nadelspitze, das in der Bereitstellung auf dem Schaft der Nadel verschiebbar ist,
- wobei das Schutzelement in der Schutzstellung durch die vorspringende Eingriffseinrichtung (18) der Nadel daran gehindert ist, über die Nadelspitze hinaus verschoben zu werden, und
- wobei zwischen Schutzelement (3) und Nadelhalter (1) ein Griffteil (6) zum Verschieben bzw. Halten des Schutzelementes (3) vorgesehen ist, und
- das Griffteil (6) das Schutzelement am proximalen Ende derart übergreift, dass es daran gehindert ist, über das Schutzelement (3) und über die Nadelspitze hinaus verschoben zu werden,
**dadurch gekennzeichnet,**
**dass** das Griffteil (6) einen Hohlraum (10) mit offenem Ende zur Aufnahme des Schutzelementes (3) aufweist, und
das Schutzelement (3) mit zwei gegenüberliegenden federnden Armen mit abgewinkelten Enden versehen ist, die die Nadelspitze in der Schutzstellung übergreifen.

2. Vorrichtung nach Anspruch 1, wobei die federnden Arme unterschiedlich lang sind.

3. Vorrichtung nach Anspruch 1 oder 2, wobei die beiden Arme sich kreuzen.

4. Vorrichtung nach Anspruch 3, wobei die abgewinkelten Ende der Arme verbreitert sind.

5. Vorrichtung nach einem der vorhergehenden Ansprüche, wobei das Griffteil (6) zylindrisch ausgebildet ist.

6. Vorrichtung nach einem der vorhergehenden Ansprüche, wobei das Griffteil (6) das Schutzelement (3) seitlich vollständig überdeckt.

7. Vorrichtung nach einem der vorhergehenden Ansprüche, wobei das Griffteil (6) einen Hohlraum (10) mit offenem distalen Ende zur Aufnahme des Schutzelementes aufweist.

## Claims

1. A protective device for an injection or infusion needle, comprising
- a needle holder (1) at the proximal end of the needle,
- a protruding engagement means (18) on the needle circumference near the needle tip,
- a protective element (3) for the needle tip, which protective element (3) is movable on the needle shaft in the ready position,
- wherein the protective element, in the protected position, is prevented by the protruding engagement means (18) of the needle from being displaced beyond the needle tip, and
- wherein a grip part (6) for moving and holding the protective element (3) is provided between protective element (3) and needle holder (1),
and
- the grip part (6) overlaps the protective element at the proximal end in such a way that it is prevented from being displaced beyond the protective element (3) and beyond the needle tip,
**characterized in that**
the grip part (6) comprises a cavity (10) with an open end for receiving the protective element (3), and
the protective element (3) comprises two opposite resilient arms with angled ends, which engage over the needle tip in the protection position.

2. The protective device according to Claim 1, wherein the resilient arms have different lengths.

3. The protective device according to Claim 1 or 2, wherein the two arms intersect.

4. The protective device according to Claim 3, wherein the angled ends of the arms are broadened.

5. The protective device according to one of the preceding claims, wherein the grip part (6) is cylindrical.

6. The protective device according to one of the preceding claims, wherein the grip part (6) completely covers the protective element (3) laterally.

7. The protective device according to one of the preceding claims, wherein the grip part (6) comprises a cavity (10) with an open distal end for receiving the protective element.

## Revendications

1. Dispositif de protection pour aiguille d'injection ou de perfusion, comprenant :
- un porte-aiguille (1) à l'extrémité proximale de l'aiguille,
- un moyen d'engagement saillant (18) sur la circonférence de l'aiguille près de la pointe de l'aiguille,
- un élément de protection (3) pour la pointe de l'aiguille qui peut être déplacé sur la tige de l'aiguille en position opérationnelle,
- le décalage de l'élément de protection (3) au-delà de la pointe de l'aiguille en position de protection étant empêché par le moyen d'engagement saillant (18) de l'aiguille et
- étant prévue entre l'élément de protection (3) et le porte-aiguille (1) une partie poignée (6) pour déplacer ou maintenir l'élément de protection (3) et
- la partie poignée (6) chevauchant l'élément de protection (3) à l'extrémité proximale de manière à l'empêcher se déplacer au-delà de l'élément de protection (3) et au-delà de la pointe de l'aiguille,
**caractérisé en ce**
**que** la partie poignée (6) présente une cavité (10) à extrémité ouverte destinée à recevoir l'élément de protection (3) et
**que** l'élément de protection (3) présent deux bras élastiques opposés à extrémités coudées qui chevauchent la pointe de l'aiguille en position de protection.

2. Dispositif selon la revendication 1, dans lequel les bras élastiques sont de longueur différente.

3. Dispositif selon la revendication 1 ou 2, dans lequel les deux bras se croisent.

4. Dispositif selon la revendication 3, dans lequel les extrémités coudées des bras sont élargies.

5. Dispositif selon une des revendications précédentes, dans lequel la partie poignée (6) a une conformation cylindrique.

6. Dispositif selon une des revendications précédentes, dans lequel la partie poignée (6) recouvre entièrement l'élément de protection (3) par côté.

7. Dispositif selon une des revendications précédentes, dans lequel la partie poignée (6) présente une cavité (10) à extrémité distale ouverte destinée à recevoir l'élément de protection.
